(19) Europäisches Patentamt
European Patent Office
Office européen des brevets

(11) Publication number: 0 151 451 B1

(12) # EUROPEAN PATENT SPECIFICATION

(45) Date of publication of patent specification: 03.08.88

(51) Int. Cl.⁴: C 07 D 213/74, A 61 K 31/44

(21) Application number: 85100876.3

(22) Date of filing: 29.01.85

(54) **4-phenylaminopyridine derivatives, process of the preparation thereof and pharmaceutical compositions containing them.**

(30) Priority: 31.01.84 LU 85193

(43) Date of publication of application: 14.08.85 Bulletin 85/33

(45) Publication of the grant of the patent: 03.08.88 Bulletin 88/31

(84) Designated Contracting States: AT BE CH DE FR GB IT LI LU NL SE

(56) References cited:
DE-A-2 054 142
DE-A-2 516 025

(73) Proprietor: BOEHRINGER MANNHEIM GMBH
Sandhofer Strasse 116
D-6800 Mannheim 31 (DE)

(72) Inventor: Ghys, Arlette
Avenue Télémaque 34
B-1190 Bruxelles (BE)
Inventor: Parmantier, Michel
Parvis Ste Alix 27
B-1150 Bruxelles (BE)
Inventor: De Suray, Jean-Marie
Rue Chesselet 5
B-5600 Sambreville (BE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention is concerned with new derivatives of 4-phenylaminopyridine, with a process for the preparation thereof and with pharmaceutical compositions containing them.

DE—A—25 16 025 describes the compound 3-isopropylcarbamylsulphonamido-4-(3′-methylphenylamino)-pyridine, also known as torasemide, which has the following structural formula:

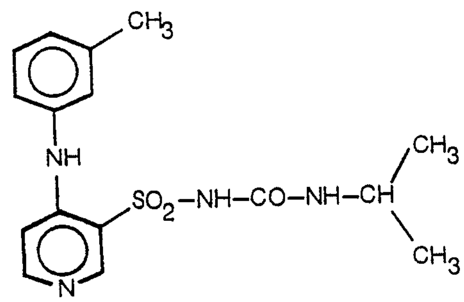

(I)

This compound has strong diuretic properties.

We have now discovered that certain derivatives of 4-phenylaminopyridine, which are related to torasemide, also possess interesting diuretic properties which have proved to be superior to those of torasemide itself.

Thus, according to the present invention, there are provided the following new derivatives of 4-phenylaminopyridine:

I 3-Isopropylcarbamylsulphonamido-4-(3′-methyl-4′-hydroxyphenylamino)-pyridine and
II 3-[1-(Hydroxymethyl)-ethylcarbamylsulphonamido]-4-(3′-methylphenylamino)-pyridine

and the pharmaceutically acceptable salts and the esters of sulfuric or glucuronic acid thereof.

The two compounds according to the present invention have been tested on rats for their diuretic activity. The tests were carried out on male Wistar rats (IFFA CREDO, Belgium) having an average body weight of 180 g. and randomly divided up into groups of three animals. The test compounds were administered by gastric intubation at a standard dosage of 50 mg./kg. of body weight in the form of an aqueous saline solution (3+ ml./kg. of body weight of a solution containing 154 millimoles of sodium chloride per litre). The comparative animals only received the aqueous saline solution (physiological saline) as a placebo. Compound I according to the present invention was also tested at a dose of 5 mg./kg. of body weight, administration being per os.

Each group of three rats being subjected to the same treatment were placed in a metabolic cage and the urine was collected over a period of 4 hours. The volume of urine was precisely measured and expressed as per kg. of body weight of the animals.

The augmentation of diuresis amongst the treated animals compared with the urinary excretion observed for the comparative animals indicates the diuretic activity of the tested compounds. The results of this test are given in the following Table I:

TABLE I

| | diuresis (ml./kg./4 hours) | |
|---|---|---|
| test compound | 50 mg./kg. p.o. | 5 mg./kg. p.o. |
| none (comparison) | 20.6 | 19.4 |
| compound I | 104.3 | 85.7 |
| compound II | 102.6 | — |
| | | — |
| torasemide | 109.3 | 75.0 |

This Table shows that at a dose of 50 mg./kg. the two compounds according to the present invention have a diuretic activity approaching that of torasemide, whereas at a dose of 5 mg./kg., at least compound I according to the present invention has a diuretic activity which is superior to that of torasemide at the same dose level.

The present invention also provides pharmaceutical compositions containing a diuretically efficacious amount of at least one compound I or II as active ingredient, together with an acceptable pharmaceutical excipient or vehicle.

Thus, the compounds according to the present invention may be used in pharmaceutical compositions containing, as active ingredient, at least one compound I or II or an acid-addition salt thereof with, for example, hydrochloric or nitric acid, or a salt thereof with sodium or potassium hydroxide, admixed with a physiologically acceptable vehicle or excipient.

The compositions according to the present invention may be administered, for example, in the form of dragees, tablets, capsules, suppositories or injection solutions, the daily dosage being from 5 to 50 mg. of active compound.

The present invention also provides a process for the preparation of compound I or II wherein a 3-sulphonamido-4-chloropyridine of the general formula:

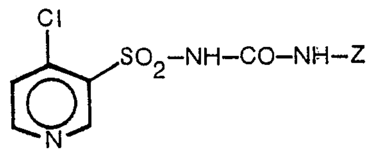

wherein Z is isopropyl or hydroxyisopropyl, is reacted with a phenylamine of the general formula:

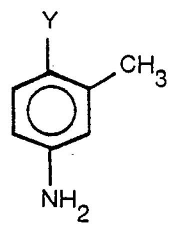

wherein Y is hydroxyl or hydrogen, whereafter the product obtained is, if desired, converted into a pharmaceutically acceptable salt.

The following Examples are given for the purpose of illustrating the present invention:

Example 1

3-Isopropylcarbamylsulphonamido-4-(3'-methyl-4'-hydroxyphenylamino)-pyridine (Compound I)

0.04 mole of 3-isopropylcarbamylsulphonamido-4-chloropyridine is reacted with 0.04 mole 4-hydroxy-3-methylaniline in 200 cc. acetone at ambient temperature for 72 hours. The precipitate obtained is filtered off, washed with acetone and then purified by dissolving in an aqueous solution of sodium carbonate, washing this alkaline solution with dichloromethane and precipitating the desired product by the addition of hydrochloric acid. The product thus obtained had a melting point of 192—193°C.

Example 2

3-[1-(Hydroxymethyl)-ethylcarbamylsulphonamido]-4-(3'-methylphenylamino)-pyridine (Compound II)

This compound is prepared in the manner described in Example 1 but using 3-methylaniline and 3-[1-(hydroxymethyl)-ethylcarbamylsulphonamido]-4-chloropyridine as starting materials.

**Claims**

1. 3-Isopropylcarbamylsulphonamido-4-(3'-methyl-4'-hydroxyphenylamino)-pyridine and 3-[1-(Hydroxymethyl)-ethylcarbamylsulphonamido]-4-(3'-methylphenylamino)-pyridine as well as the pharmaceutically acceptable salts and the esters of sulphuric or glucuronic acid thereof.

2. Process for the preparation of 3-Isopropylcarbamylsulphonamido-4-(3'-methyl-4'-hydroxyphenyl-amino)-pyridine according to claim 1, wherein 3-isopropylcarbamylsulphonamido-4-chloropyridine is reacted with 4-hydroxy-3-methylaniline whereafter, if desired, the product obtained is converted into a pharmaceutically acceptable salt or the above mentioned ester.

3. Process for the preparation of 3-[1-(Hydroxymethyl)-ethylcarbamylsulphonamido]-4-(3'-methylphenylamino)-pyridine according to claim 1, wherein 3-[1-(Hydroxymethyl)-ethylcarbamylsulphonamido]-4-chloropyridine is reacted with 3-methylaniline, whereafter, if desired, the product obtained is converted into a pharmaceutically acceptable salt or the above mentioned ester.

4. Pharmaceutical compositions comprising at least one compound according to claim 1 in a diuretically efficacious amount as active component, together with a pharmaceutically acceptable excipient or vehicle.

5. Use of at least one compound according to claim 1 for the manufacture of a medicament with diuretic properties.

**Patentansprüche**

1. 3-Isopropylcarbamylsulphonamido-4-(3'-methyl-4'-hydroxyphenylamino)-pyridin und 3-[1-(Hydroxymethyl)-ethylcarbamylsulphonamido]-4-(3'-methylphenylamino)-pyridin sowie ihre pharmakologisch verträglichen Salze und Schwefelsäure- und Glucuronsäureester.

2. Verfahren zur Herstellung von 3-Isopropylcarbamylsulphonamido-4-(3'-methyl-4'-hydroxyphenylamino)-pyridin gemäß Anspruch 1, dadurch gekennzeichnet, daß 3-Isopropylcarbamylsulphonamido-4-chloropyridin mit 4-Hydroxy-3-methylanilin umgesetzt wird, und anschließend gewünschtenfalls das erhaltene Produkt in ein pharmazeutisch verträgliches Salz oder den oben aufgeführten Ester überführt.

3. Verfahren zur Herstellung von 3-[1-(Hydroxymethyl)-ethylcarbamylsulphonamido]-4-(3'-methylphenylamino)-pyridin gemäß Anspruch 1, dadurch gekennzeichnet, daß man 3-[1-Hydroxymethyl)-ethylcarbamylsulphonamido]-4-chlorpyridin mit 3-Methylanilin umsetzt, und anschließend gewünschtenfalls das erhaltene Produkt in ein pharmazeutisch verträgliches Salz oder den oben aufgeführten Ester überführt.

4. Arzneimittel bestehend aus mindestens einer Verbindung gemäß Anspruch 1 in einer diurestisch wirksamen Menge als aktiver Bestandsteil zusammen mit einem pharmazeutisch verträglichen Hilfs- oder Trägerstoff.

5. Verwendung von mindestens einer Verbindung gemäß Anspruch 1 zur Herstellung eines Arzneimittels mit diuretischen Eigenschaften.

**Revendications**

1. 3-Isopropylcarbamylsulphonamido-4-(3'-méthyl-4'-hydroxyphénylamino)-pyridine et 3-[1-(Hydroxyméthyl)-éthylcarbamylsulphonamido]-4-(3'-méthylphénylamino)-pyridine ainsi que leurs sels pharmaceutiquement acceptables et ses esters formés avec l'acide sulfurique ou l'acide glucuronique.

2. Procédé pour la préparation de la 3-isopropylcarbamylsulphonamido-4-(3'-méthyl-4'-hydroxyphénylamino)-pyridine selon la revendication 1, dans lequel on fait réagir la 3-isopropylcarbamylsulphonamido-4-chloropyridine avec la 4-hydroxy-3-méthylaniline, après quoi, si nécessaire, on transforme le produit obtenu en un sel pharmaceutiquement acceptable ou en l'ester mentionné ci-dessus.

3. Procédé pour la préparation de la 3-[1-(hydroxyméthyl)-éthylcarbamylsulphonamido]-4-(3'-méthylphénylamino)-pyridine selon la revendication 1, dans lequel on fait réagir la 3-[1-hydroxyméthyl)-éthylcarbamylsulphonamido]-4-chloropyridine avec la 3-méthylaniline, après quoi, si nécessaire, on transforme le produit obtenu en un sel pharmaceutiquement acceptable ou en l'ester mentionné ci-dessus.

4. Compositions pharmaceutiques comprenant au moins un composé selon la revendication 1, comme composant actif, en une quantité efficace du point de vue diurétique, en commun avec un excipient ou véhicule pharmaceutiquement acceptable.

5. Utilisation d'au moins un composé selon la revendication 1, pour la fabrication d'un médicament possédant des propriétés diurétiques.